(19) Europäisches Patentamt
European Patent Office
Office européen des brevets

(11) Publication number: **0 175 269 B1**

(12) **EUROPEAN PATENT SPECIFICATION**

(45) Date of publication of patent specification: **06.03.91**

(51) Int. Cl.⁵: **C07K 1/00**, C07K 3/00, G01N 33/53, G01N 33/94, A61K 39/385, G01N 33/543

(21) Application number: **85111468.6**

(22) Date of filing: **11.09.85**

# (54) Immunogen conjugates and the use thereof in a dihydropyridine assay.

(30) Priority: **21.09.84 US 653699**

(43) Date of publication of application: **26.03.86 Bulletin 86/13**

(45) Publication of the grant of the patent: **06.03.91 Bulletin 91/10**

(84) Designated Contracting States: **AT CH DE FR GB IT LI NL SE**

(56) References cited:
**EP-A- 0 052 921**
**EP-A- 0 117 888**
**US-A- 4 329 502**

(73) Proprietor: **UNIVERSITY OF IOWA RESEARCH FOUNDATION**
**203, Gilmore Hall**
**Iowa City, IA 52242(US)**

(72) Inventor: **Campbell, Kevin P.**
**1216 Wade Street**
**Iowa City Iowa 52240(US)**

(74) Representative: **Kedinger, Jean-Paul et al**
**c/o Cabinet Malemont 42, avenue du Président Wilson**
**F-75116 Paris(FR)**

**EP 0 175 269 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

$Ca^{2+}$ channel blockers are widely used for the treatment of various cardiovascular disorders. Nifedipine, nitrendipine, nisoldipine, nimodipine and related 1,4-dihydropyridines (hereafter alternatively referred to as DHPs) are the most potent class of $Ca^{2+}$ channel blockers. Radioreceptor and high performance liquid chromatography assays are available for the measurement of these agents in human serum, however, such techniques are cumbersome and costly and are not readily amenable to large-scale use.

The present invention provides immunogen conjugates comprising a DHP derivative coupled to immunogenic carrier materials and antibodies prepared against such immunogen conjugates. Such antibodies are useful in immunoassays for determining DHPs in biological fluids, thus filling the need for a rapid and sensitive assay for large-scale clinical use.

### DESCRIPTION OF PERTINENT ART

Janis, et al ( J. Clin. Pharmacol. , 1983; 23 : 266-273) describe the development of radioreceptor and high performance liquid chromatographic assays for the calcium channel antagonist nitrendipine in serum samples. Krol, et al, J. Chromatography , 1983 (in press) teach the use of gas and liquid chromatographic analysis of the calcium channel antagonist nimodipine in blood, plasma and cerebrospinal fluid.

Neither of these references describe or suggest the immunogen conjugates or the antibodies raised thereto disclosed in the present invention nor the use of said antibodies in immunoassays for 1,4-dihydropyridines.

### SUMMARY OF THE INVENTION

The present invention discloses 1,4-dihydropyridine immunogen conjugates of immunogenic carrier materials coupled to a 1,4-dihydropyridine derivative. The immunogen conjugates disclosed are of the general formula:

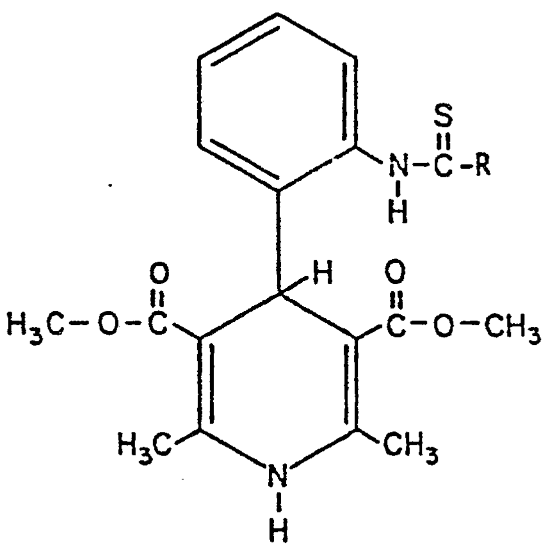

I

wherein R is a protein or polypeptide.

Antibodies prepared against the immunogen conjugates of formula I and the use thereof in immunoassays are also taught.

Also disclosed is an affinity column useful for the purification of antibodies specific to 1,4-dihydropyridines from antiserum. Said affinity column is composed of 1,4-dihydropyridine-containing immunoadsorbent conjugates which are represented by the general formula:

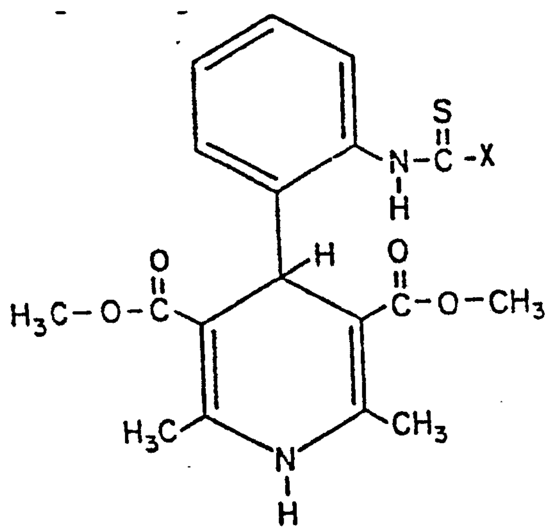

wherein X is an insoluble support matrix having a free available amino functional group.

The immunoadsorbent conjugates are used for purifying antibodies specific for 1,4-dihydrpyridines from an antiserum containing said antibodies. The purification method includes the steps of: (a) incubating a mixture of said antiserum with a 1,4-dihydropyridine-containing immunoadsorbent conjugate thereby binding said antibodies to said conjugates; (b) washing the mixture of (a) to remove nonspecifically bound material; and (c) dissociating said antibodies from said conjugate by incubating the mixture of (a) at from about 0° C to about 10° C with a dissociative reagent thereby enhancing recovery of functional antibodies and isolating therefrom said antibodies.

## DETAILED DESCRIPTION OF THE INVENTION

The DHP derivative which is used in the preparation of the immunogen conjugates is an affinity analog of nifedipine and is chemically, 1,4-dihydro-2,6-dimethyl-4-(2-isothiocyantophenyl)--3,5-pyridinecarboxylic acid dimethyl ester (hereafter referred to as nifedipine-isothiocyanate) and is represented by the formula:

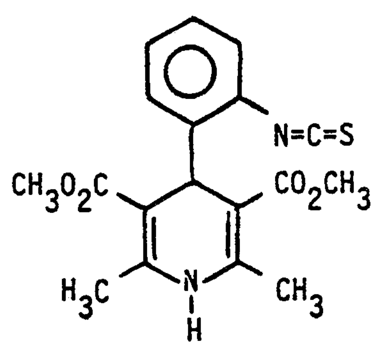

Nifedipine-isothiocyanate is commercially available from New England Nuclear Corp. or may be prepared as described by Venter, et al in The Journal of Biological Chemistry , Vol. 258 (15), 9344-9348 (1983), which is incorporated herein by reference.

The nifedipine-isothiocyanate analog is covalently coupled to an immunogenic carrier material as described hereafter. The immunogenic carrier material may be a protein or polypeptide having available amino functional groups for coupling to the nifedipine-isothiocyanate derivative. When the immunogenic carrier is a protein-aceous material, said carrier is preferably incubated with nifedipine-isothiocyanate present in a sufficiently high concentration to form those immunogen conjugates having a ratio of nifedipine-isothiocyanate to carrier of about at least one to one. A sufficiently high concentration of nifedipine-isothiocyanate can be maintained by the inclusion of a sufficient amount of ethanol to maintain the solubility of the nifedipine-isothiocyanate during the incubation.

For the most part, immunogenic proteins and polypeptides will have molecular weights between 4,000 and 10,000,000 preferably greater than 15,000, and more usually greater than 50,000. Generally, proteins

taken from one animal species will be immunogenic when introduced into the blood stream of another species. Particularly useful proteins are albumins, globulins, enzymes, hemocyanins, glutelins, proteins having significant nonproteinaceous constituents, e.g., glycoproteins, and the like. Further reference for the state-of-the-art concerning conventional immunogenic carrier materials and techniques for coupling haptens thereto may be had to the following (each of which are incorporated herein by reference): Parker, *Radioimmunoassay of Biologically Active Compounds*, Prentice-Hall (Englewood Cliffs, New Jersey, U.S.A., 1976); Butler, *J. Immunol. Meth.*, 7:1-24 (1974); Weinryb and Shroff, *Drug Metab. Rev.*, 10:271-283 (1975); Broughton and Strong, *Clin. Chem.*, 22:726-732 (1976); and Playfair, et al, *Br. Med. Bull.*, 30:24-31 (1974). Preferred proteins for use as immunogenic carrier materials are bovine serum albumin, casein, ovalbumin and keyhole limpet hemocyanin. Most particularly preferred is keyhole limpet hemocyanin.

Preparation of specific antibodies using the present immunogen conjugates may follow any conventional technique. Numerous texts are available describing the fundamental aspects of inducing antibody formation. For example, see Parker, *Radioimmunoassay of Biologically Active Compounds*, Prentice-Hall (Englewood Cliffs, New Jersey, U.S.A., 1976) which is incorporated herein by reference. In the usual case, a host animal such as a rabbit, goat, mouse, guinea pig, sheep or horse is injected at one or more of a variety of sites with the immunogen conjugate, normally in admixture with an adjuvant. Further injections are made at the same site or different sites at regular or irregular intervals thereafter with bleedings being taken to assess antibody titer until it is determined that optimal titer has been reached. The host animal is bled to yield a suitable volume of specific antiserum. The antiserum itself may then be used in performing the actual assays or purification steps may be taken to remove undesired material such as nonspecific antibodies and the like. Such purification techniques include the preparation of an IgG fraction from the antiserum using ammonium sulfate or by affinity purification from an insoluble 1,4-dihydropyridine-containing immunoadsorbent conjugate such as, for example, that prepared as described hereafter from nifedipine-isothiocyanate and AH-Sepharose 4B or nifedipine-isothiocyanate and lysine-Sepharose 4B. The 1,4-dihydropyridine-containing immunoadsorbent conjugate is then used to purify antibodies from an antiserum containing the antibodies. The antiserum is incubated with the desired immunoadsorbent conjugate (preferably a conjugate prepared from nifedipine-isothiocyanate and AH-Sepharose 4B or nifedipine-isothiocyanate and lysine-Sepharose 4B) which serves to bind the 1,4-dihydropyridine-specific antibodies to the conjugate. This mixture is then washed to remove non-specifically bound materials with, for example, phosphate buffered saline or a sodium chloride solution. Following the washing, the mixture is then incubated with a solution capable of dissociating the bound antibodies from the conjugate. This solution may be any such solution capable of affecting such dissociation while still serving to enhance the recovery of functional antibodies. Preferred for such dissociation is an alkaline solution containing sodium chloride and dioxane. Particularly preferred is a solution of about pH 11, containing about 1 M sodium chloride, about 50 mM diethylamine and about 10 percent dioxane. The incubation may be carried out at about 0°C to about 10°C, preferably at about 0°C to about 4°C. Isolation of the dissociated antibodies is then achieved by conventional techniques.

Initial testing for the presence of the DHP-specific antibody may be carried out using an indirect immuno-peroxidase staining of the immunogen conjugates as follows. Control (unlabeled) proteins and nifedipine-protein immunogen conjugates (different from the carrier nifedipine-protein conjugate used for immunization), are either dotted onto nitrocellulose paper or transferred following SDS gel electrophoresis onto nitrocellulose paper. The nitrocellulose blot is then incubated with the test antiserum followed by a secondary antibody conjugated to peroxidase and then developed with a peroxidase substrate. Positive antisera will react with the nifedipine-protein conjugate but not with the unlabeled protein.

The antibodies prepared against the immunogen conjugates have been found to provide excellent specificity. Inactive metabolites of nitrendipine as well as structurally unrelated calcium channel blockers verapamil and diltiazem do not cross-react with said antibodies. Further, one skilled in the art will readily appreciate that monoclonal antibodies for specific 1,4-dihydropyridines may be prepared by standard techniques such as the following. Female BALB/C mice are immunized with nifedipine isothiocyanate-keyhole limpet hemocyanin immunogen conjugates and then boosted three times with the same conjugates after four weeks. Serum samples are tested using radioimmunoassay and indirect immunostaining assays to insure a high titer of anti-1,4-dihydropyridine antibodies. Fusions are performed with an equal number of spleen cells from an immunized mouse and myeloma cells (NS-1) using polyethylene glycol. Cells from each fusion are plated into twenty 96-well microtiter plates. Hybridoma colonies producing anti-1,4-dihydropyridine antibodies are selected using an indirect immunoperoxidase dot assay against nifidipine bovine serum albumin.

Preferably, the antibodies derived from the immunogen conjugates are to be used in a radioimmunoassay (RIA) for determining the quantity of DHP present in a biological sample. However, it is well-known that

such antibodies are also required in various other assays. These assays, which are contemplated equivalents to the radioimmunoassay described hereinafter, include heterogeneous and homogeneous enzyme-labeled binding assays, fluorescence-labeled binding assays, and chemiluminescence-labeled binding assays. Such assays may be used for determining the concentration on any dihydropyridine in a biological sample such as nitrendipine, nifedipine, nisoldipine, nimodipine and the like.

The RIA for the 1,4-dihydropyridines is based on the competition between 1,4-dihydropyridines in a biological sample and a fixed quantity of radiolabeled-1,4-dihydropyridine such as radiolabeled-nitrendipine (i.e., 2,6-dimethyl-3-carboethoxy-5-carbomethoxy-4-(3-nitrophenyl)-1,4 dihydropyridine) to an antibody which has a high specificity and affinity for the 1,4-dihydropyridines. The radiolabeled nitrendipine can be labeled in any manner well-known in the art with any suitable radionuclide. A listing of the radionuclides which are now conventionally in use in reagents and which may be used in this invention are listed in the index of radionuclides found on page 81 of the 1978 edition of the Catalogue of the New England Nuclear Corporation, Boston, Mass, U.S.A., (New England Nuclear, 1977) incorporated herein by reference. Among radionuclides which may be utilized include the following: hydrogen-3 (tritium) and the radioisotopes of iodine ($^{123}I$, $^{124}I$, $^{125}I$, $^{126}I$, $^{128}I$, $^{130}I$, $^{131}I$ and $^{132}I$) with $^{3}H$ being preferred from considerations of availability, half life and specific activity and/or the ability of these to be readily detected using a conventional liquid scintillation counter.

The RIA can be performed by any manner known in the art. For example, the antiserum containing the antibody prepared against the immunogen conjugate (or alternatively the Ig fraction obtained by ammonium sulfate purification or the affinity purified antibody) can be diluted to a suitable concentration with a buffer containing the radiolabeled nitrendipine (or other radiolabeled 1,4-dihydropyridine) and incubated under suitable conditions until equilibrium is reached with either the biological sample to be determined or a standard solution used for preparing a standard curve. Separation of the 1,4-dihydropyridine bound to antibody from the free 1,4-dihydropyridine is affected by well-known procedures such as by the use of dextran-coated charcoal, Protein A Sepharose beads, the double antibody method and the like. Preferably, the separation is achieved using dextran-coated charcoal. The radioactivity of one of the separated substances is counted by, for example, a weld-type scintillation counter and a standard curve is prepared based on the value obtained for standard solutions assayed by the same technique. The DHP level in the biological sample being tested is then determined based on that standard curve.

The following examples are set forth as a means of illustrating the present invention and are not to be construed as a limitation thereon.

## Example 1

### Preparation of Immunogen Conjugates

Nifedipine-isothiocyanate (1,4-dihydro-2,6-dimethyl-4-(2-isothiocyanatophenyl)-3,5-pyridinedicarboxylic acid dimethyl ester) obtained from New England Nuclear was coupled to bovine serum albumin as follows. Bovine serum albumin (10 mg) was dissolved in 10 ml of 100 mM sodium bicarbonate (pH 9.0) and then incubated with 0.6 mg of nifedipine-isothiocyanate (dissolved in ethanol to make the final concentration of ethanol in the incubation medium 0.65 percent) for 24 hours at room temperature with shaking in the dark which thus formed the desired nifedipine-isothiocyanate bovine serum albumin immunogen conjugate. The formation of said conjugate was monitored by including [$^{3}$H]nifedipine-isothiocyanate in the preparation of the bovine serum albumin-nifedipine conjugate. Bovine serum albumin-[$^{3}$H]nifedipine conjugate (10-40 micrograms) was separated from free nifedipine by SDS-polyacrylamide gel electrophoresis and [$^{3}$H]-nifedipine incorporation was detected by fluorography or gel slice analysis. Gel slices (2.5 mm) were digested overnight at 45°C in 0.8 ml of 30% hydrogen peroxide before liquid scintillation fluid was added. Radioactivity was measured using a Beckman LS8100 counter.

Nifedipine-isothiocyanate was also coupled to casein, ovalbumin and keyhole limpet hemocyanin using the procedures described above. An alternate procedure for the coupling of nifedipine-isothiocyanate to various proteins (i.e., bovine serum albumin, ovalbumin, casein and keyhole limpet hemocyanin) was to dissolve the protein (20 mg) in 2 ml of 100 mM sodium bicarbonate (pH 9.0) and then incubate with 0.8 mg of nifedipine-isothiocyanate (dissolved in ethanol to make the final concentration of ethanol in the incubation medium 38 percent) for 24 hours at 37°C with shaking in the dark. This alternate procedure is useful for producing nifedipine conjugates at high protein concentrations.

Example 2

Production and Isolation of Antibodies

In order to raise antibodies against the 1,4 dihydropyridines, a bovine serum albumin-nifedipine isothiocyanate immunogen conjugate was used directly for immunization or was cross-linked with 0.04% glutaraldehyde for eight hours at room temperature prior to immunization of rabbits. In either case, the immunogen conjugate was emulsified in an equal volume of Freund's complete adjuvant.

New Zealand white rabbits were bled to obtain pre-immune sera and were then immunized intramuscularly and intradermally at multiple sites along the back and legs. Rabbits received 0.5 milligram (mg) of bovine serum albumin-nifedipine isothiocyanate immunogen conjugate in Freund's complete adjuvant on day 0 followed by 0.5 mg insults in Freund's incomplete adjuvant every 2-3 weeks. Rabbits were bled after their third immunization every two weeks. Blood was withdrawn from the ear vein, allowed to clot overnight at 4° C, and serum was isolated by centrifugation. In order to reduce the antibodies produced to bovine serum albumin, subsequent immunizations were carried out with keyhole limpet hemocyanin-nifedipine isothiocyanate immunogen conjugates. Using the procedures described above, casein-nifedipine isothiocuprate, ovalbumin-nifedipine isothiocyanate and keyhole limpet hemocyanin-nifedipine isothiocyanate immunogen conjugates were also used to immunize rabbits and isolate specific antibodies produced to the conjugates. The keyhole limpet hemocyanin-nifedipine isothiocyanate immunogen conjugate was mixed with an equal volume of aluminum hydroxide and phosphate buffered saline and stirred for 1 hour at 4° C and then emulsified in an equal volume of Freund's complete adjuvant prior to use in the immunizations.

All of the above procedures were successful in obtaining high affinity 1,4 dihydropyridine-specific antibodies in rabbits. Keyhole limpet hemocyanin produced a response in the shortest time (4 weeks) and produced the highest titer antiserum of 1,4 dihydropyridine-specific antibodies. Bovine serum albumin-nifedipine isothiocyanate immunogen conjugate and the keyhole limpet hemocyanin-nifedipine isothiocyanate immunogen conjugate were also used in the immunization of sheep to produce anti-1,4-dihydropyridine antibodies.

1,4 dihydropyridine-specific antibody (obtained as described in Example 2) was partially purified from antisera by a 50% ammonium sulfate precipitation in the cold for 24 hours. The precipitated IgG fraction was then resuspended in phosphate buffered saline and dialysed against phosphate buffered saline in the cold for 24 hours.

Example 3

Preparation of Nifedipine-Sepharose Affinity Column

Nifedipine-isothiocyanate obtained from New England Nuclear was coupled to AH-Sepharose 4B obtained from Pharmacia as follows. AH-Sepharose 4B (5 g) was resuspended and washed three times in 80 ml of 20 mM sodium bicarbonate (pH 9.0) and then resuspended in 100 ml of sodium bicarbonate (pH 8.0). Nifedipine-isothiocyanate (10 mg) was dissolved in 10 ml of 95% ethanol and 5 $\mu$l of [$^3$H]nifedipine-isothiocyanate (83 Ci/mmole) was added as tracer. AH-Sepharose 4B beads (25 ml) nifedipine-isothiocyante (0.1 to 8 ml) and at least 10 percent ethanol were then incubated at 37° C overnight. The beads were then washed with 40 ml of the following buffers: 0.15 M NaCl, 10 mM TRIS, pH 7.4; 1 M TRIS pH 7.5; phosphate buffered saline with 0.05% Tween-20 and finally four times with 0.15 M NaCl, 10 mM TRIS, pH 7.4. The nifedipine-Sepharose 4B immunoadsorbent conjugate was then resuspended in 20 ml of 0.15 M NaCl, 10 mM TRIS pH 7.4. Percent of nifedipine-isothiocyanate coupled to AH-Sepharose was measured by counting a sample of the nifedipine-Sepharose 4B immunoadsorbent conjugate in a LS 8100 liquid scintillation counter.

Example 4

Affinity Purification of Nifedipine-Specific Antibodies

The nifedipine-Sepharose 4B affinity column (1.0 ml packed column) was prepared as described in Example 3 and was incubated with 100 ml sheep anti-nifedipine serum for 24 hours at room temperature, with gentle shaking. The affinity column was then washed extensively with phosphate buffered saline and then 1 M NaCl. The nifedipine-specific antibodies were then eluted by incubation at about 0-4° C with 1 M NaCl, 50 mM diethylamine (pH 11.5) and 10% dioxane. The eluted antibodies were quickly placed on a G-25 M Sepharose column for desalting and were recovered in phosphate buffered saline thereby returning the antibodies to a neutral pH.

Example 5

Immunoblot Characterization of the Prepared Antibodies

Unlabeled and nifedipine-labeled proteins were dotted onto nitrocellulose paper or transferred onto nitrocellulose paper following SDS polyacrylamide gel electrophoresis. Nitrocellulose blots were blocked with 0.05% Tween-20 or 3% bovine serum albumin in phosphate buffered saline for 1 hour at room temperature. Blots were then incubated for 1 hour at room temperature with a 1:10 to 1:1000 dilution of test antisera in the presence of blocking agent. Following washing, the blots were incubated for 1 hour with a 1:1000 dilution of goat antirabbit IgG peroxidase conjugate. Blots were then developed with 4-chloro-1-naphthol following washing. Specificity for the staining was shown using the unlabeled proteins as controls and/or by including $10^{-7}$ M nitrendipine in the primary incubation with the test antisera.

The experimental data indicated that the prepared antibody recognized nifedipine coupled to carbonic anhydrase while it did not react with unlabeled carbonic anhydrase.

Example 6

1,4-Dihydropyridine Radioimmunoassay

The radioimmunoassay of a sample containing an unknown amount of 1,4-dihydropyridine was performed using 1.5 ml Eppendorf tubes in the dark. 1,4-dihydropyridine standards were prepared by serial dilution in 0.1% bovine serum albumin in saline and stored at 4° C in the dark. 1,4-dihydropyridine-specific antibodies were prepared as described in Example 2 or were affinity purified as described in Example 4. A titer test was then performed with the 1,4-dihydropyridine-specific antibody using a fixed amount (for example 20 pM) of [$^3$H]nitrendipine (obtained from New England Nuclear) to determine the dilution needed for binding 50% of the added [$^3$H]nitrendipine.

Each assay tube contained a 0.1 ml aliquot of sample or standard and 1 ml of the following assay medium: 150 mM NaC1, 10 mM TRIS (pH 7,.2), 0.1% gelatin, 20 pM [$^3$H]nitrendipine and the appropriate amount of antibody as determined by the titer test above. After a 1 hour incubation in the dark at room temperature, the assay tubes were transferred to an ice water bath for 10 min. Separation of free and antibody bound [$^3$H]nitrendipine was achieved by the addition of 0.2 ml of dextran-coated charcoal. After an additional 12 minutes in the ice bath, the assay tubes were centrifuged for 15 minutes in an Eppendorf centrifuge. A 0.65 ml sample of the clear supernatant was then added to 10 ml of liquid scintillation fluid and counted in a Beckman LS8100 liquid scintillation counter. A standard curve was constructed by plotting the average percent $B/B_O$ (where B is the [$^3$H]nitrendipine bound in the presence of an unknown amount of unlabeled 1,4-dihydropyridine and $B_O$ is the [$^3$H]nitrendipine bound in the absence of unlabeled 1,4-dihydropyridine) of the triplicate values of each standard on the linear axis of semilogarithmic graph paper as a function of the concentration of the standards in picograms per milliliter. The average percent $B/B_O$ of the triplicate values of each sample and control was determine and the 1,4-dihydropyridine concentration from the sample or control percent $B/B_O$ value from the standard curve was also determined. The nitrendipine net counts per minute (cpm) and percent $B/B_O$ values are set forth in Table I. Similar standard curves were prepared for other 1,4-dihydropyridines.

TABLE I

| Nitrendipine Standard (picograms/ml) | Net CPM | % B/$B_o$ |
|---|---|---|
| 0.0 | 1747 | 100.0 |
| 18.0 | 1492 | 85.4 |
| 36.0 | 1406 | 80.5 |
| 72.0 | 1024 | 58.6 |
| 180.0 | 673 | 38.5 |
| 360.0 | 482 | 27.6 |
| 720.0 | 346 | 19.8 |
| 1800.0 | 225 | 12.9 |

[a]Concentration of nitrendipine standard in the final assay mixture.

## Claims

1. 1,4-dihydropyridine immunogen conjugates of the formula:

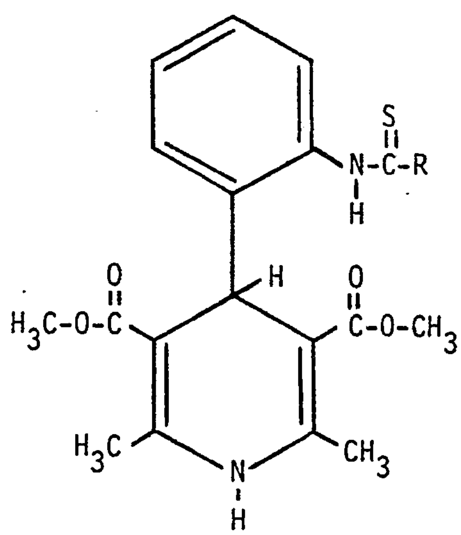

wherein R is a protein or polypeptide.

2. The immunogen conjugate of Claim 1 wherein the protein is bovine serum albumin, casein, ovalbumin or keyhole limpet hemocyanin.

3. The immunogen conjugate of Claim 1 or 2 wherein the protein is keyhole limpet hemocyanin.

4. An antibody prepared against the immunogen conjugate of any of the Claims 1 to 3.

5. A method of detecting the presence of 1,4-dihydropyridines in a biological sample which comprises reacting said biological sample containing an unknown amount of a l,4-dihydropyridine, a fixed amount of radiolabeled 1,4-dihydropyridine and a predetermined amount of the antibody of Claim 4 having high specificity to said 1,4-dihydropyridine and thereafter separating the resulting antigen-antibody complex from unbound antigen and measuring the radioactive content of either the complex or the unbound antigen.

6. The method of Claim 5 wherein the radiolabeled 1,4-dihydropyridine is [$^3$H] nitrendipine.

## Revendications

1. Conjugués de 1,4-dihydropyridine immunogène, de formule:

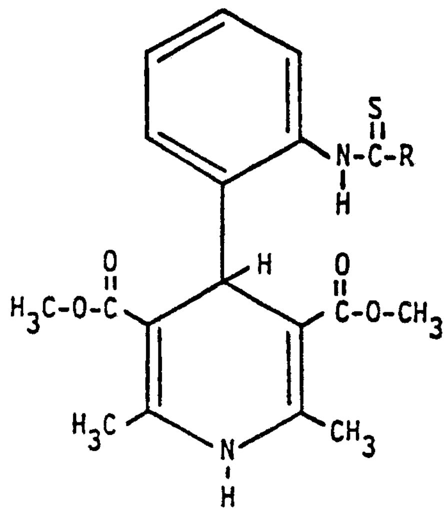

dans laquelle R représente une protéine ou un polypeptide.

2. Conjugué d'immunogène selon la revendication 1, dans lequel la protéine est de la sérum albumine de bovin, de la caséine, de l'ovalbumine ou de l'hémocyanine de mollusque (en forme de trou de clé de serrure).

3. Conjugué d'immunogène selon la revendication 1 ou 2, dans lequel la protéine est de la l'hémocyanine de mollusque.

4. Anticorps préparé contre le conjugué d'immunogène selon l'une quelconque des revendications 1 à 3.

5. Procédé pour déceler la présence de 1,4-dihydropyridines dans un échantillon biologique, ce procédé comprenant la réaction dudit échantillon biologique, contenant une quantité inconnue d'une 1,4-dihydropyridine, une quantité déterminée d'une 1,4-dihydropyridine radiomarquée et une quantité prédéterminée de l'anticorps selon la revendication 4, ayant une grande spécificité à l'égard de ladite 1,4-dihydropyridine, puis la séparation du complexe résultant antigène/anticorps d'avec l'antigène non lié et la mesure de la teneur en matière radioactive du complexe ou bien de l'antigène non lié.

6. Procédé selon la revendication 5, dans lequel la 1,4-dihydropyridine radiomarquée est de la [$^3$H]-nitrendipine (ou nitrendipine marquée par $^3$H).

## Ansprüche

1. 1,4-Dihydropyridin-Immunogenkonjugate der Formel

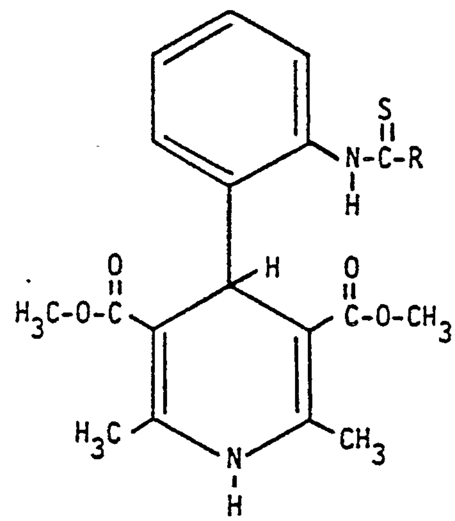

worin R ein Protein oder Polypeptid ist.

2. Immunogenkonjugat gemäss Anspruch 1, worin das Protein Rinderserumalbumin, Kasein, Ovalbumin oder Schlüssellochschnecken-Hämocyanin ist.

3. Immunogenkonjugat gemäss Ansprüchen 1 oder 2, worin das Protein Schlüssellochschnecken-Hämocyanin ist.

4. Antikörper, der gegen das Immunogenkonjugat gemäss Ansprüchen 1 bis 3 gerichtet ist.

5. Verfahren zum Nachweis der Anwesenheit von 1,4-Dihydropyridinen in einer biologischen Probe, welches das Umsetzen der biologischen Probe, die eine unbekannte Menge eines 1,4-Dihydropyridins, eine festgelegte Menge eines radiomarkierten 1,4-Dihydropyridins und eine vorgegebene Menge des Antikörpers gemäss Anspruch 4 mit hoher Spezifität für das 1,4-Dihydropyridin enthält, und danach das Abtrennen des entstehenden Antigen-Antikörper-Komplexes von ungebundenem Antigen und das Bestimmen des radioaktiven Gehaltes entweder des Komplexes oder des ungebundenen Antigens umfasst.

6. Verfahren gemäss Anspruch 5, worin das radiomarkierte 1,4-Dihydropyridin [$^3$H]Nitrendipin ist.